(19) Europäisches Patentamt / European Patent Office / Office européen des brevets

(11) **EP 2 640 261 B1**

## (12) FASCICULE DE BREVET EUROPEEN

(45) Date de publication et mention
de la délivrance du brevet:
**22.10.2014 Bulletin 2014/43**

(21) Numéro de dépôt: **11797105.1**

(22) Date de dépôt: **17.11.2011**

(51) Int Cl.:
*A61B 5/024* (2006.01)   *A63B 24/00* (2006.01)
*G01C 21/00* (2006.01)   *A61B 5/11* (2006.01)

(86) Numéro de dépôt international:
**PCT/FR2011/052683**

(87) Numéro de publication internationale:
**WO 2012/066249 (24.05.2012 Gazette 2012/21)**

(54) **DISPOSITIF D'ASSISTANCE À L'EFFORT PHYSIQUE PERMETTANT DE PRÉVOIR L'ÉVOLUTION D'UN PARAMÈTRE PHYSIOLOGIQUE EN FONCTION D'UN ITINÉRAIRE**

TRAININGSHILFSVORRICHTUNG ZUR VORHERSAGE DER VERÄNDERUNG EINES PHYSIOLOGISCHEN PARAMETERS ALS FUNKTION EINER ROUTE

EXERCISE-ASSISTING DEVICE FOR FORECASTING THE CHANGE IN A PHYSIOLOGICAL PARAMETER AS A FUNCTION OF AN ITINERARY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.11.2010 FR 1004509**

(43) Date de publication de la demande:
**25.09.2013 Bulletin 2013/39**

(73) Titulaire: **Centre National d'Etudes Spatiales (CNES)**
**75001 Paris (FR)**

(72) Inventeur: **ROUQUETTE, Sébastien**
**31320 CASTANET-TOLOSAN (FR)**

(74) Mandataire: **Cabinet BARRE LAFORGUE & associés**
**35, rue Lancefoc**
**31000 Toulouse (FR)**

(56) Documents cités:
**EP-A1- 1 691 170**     **US-A1- 2004 046 692**
**US-A1- 2008 009 275**     **US-A1- 2008 109 158**
**US-B1- 7 534 206**

**Description**

L'invention concerne un dispositif permettant d'assister l'effort physique d'un être humain ou d'un animal sur un itinéraire prédéterminé. Dans toute la suite on désigne par « sujet » tout être humain ou animal sur lequel un tel dispositif est utilisé pour prévoir au moins l'un de ses paramètres physiologiques.

L'utilisateur d'un tel dispositif peut être le sujet lui-même (couramment lorsque le sujet est un être humain), ou bien une personne qui n'est pas le sujet (cas d'un utilisateur humain qui souhaite surveiller l'effort d'un animal).

Dans de nombreuses situations il est avantageux de pouvoir assister et notamment surveiller et prévoir l'effort physique d'un sujet.

Un effort physique est fourni dans de nombreuses situations. Par exemple un sujet en rééducation suite à un traumatisme ou à une opération peut avoir à effectuer des exercices physiques ne demandant qu'un effort modéré. Il est donc avantageux de pouvoir assister et prévenir un effort jugé trop important pour un sujet en phase de rééducation. A contrario, des situations où l'effort physique à fournir est de haut niveau, comme lors d'entraînements ou de compétitions de sport demandent un effort physique important qu'on peut souhaiter surveiller et/ou optimiser. En outre, l'effort physique peut être constitué en grande partie par un stress, par exemple lors d'un raid en planeur ou d'une course automobile.

L'organisme d'un sujet régule, au maximum de ses capacités, ses paramètres physiologiques dans le but de se protéger. Ainsi la variation de ces paramètres physiologiques et leur évolution reflètent l'état physiologique et physique d'un sujet. Par exemple, la saturation en dioxygène du sang est un très bon indicateur de l'état physique d'un sujet.

La fréquence cardiaque (FC) est un autre paramètre physiologique très fiable quant à l'état de l'organisme d'un sujet. Ainsi une fréquence cardiaque plus élevée indique généralement un niveau d'hypoxie plus avancé.

On connaît à ce sujet le document « A dynamic heart rate model for training optimization in cycling (P83) », Ankang Le, Thomas Jaitner, Frank Tobias, Lothar Litz, in The Engineering and Sport 7, ed. Springer Paris, qui décrit un dispositif permettant de collecter et prévoir, avant une session d'entraînement, le rythme cardiaque de cyclistes sur des vélos d'intérieur qui imposent au cycliste une puissance donnée.

Cependant, le sujet d'un tel dispositif ne peut pas se déplacer sur un itinéraire réel, par définition bien plus complexe que toute simulation imposée par un vélo d'intérieur.

De plus, il est donc impossible pour l'utilisateur du dispositif de faire des choix en temps réel sur l'allure du sujet, car il ne peut pas réévaluer régulièrement quels seront les efforts futurs à fournir par le sujet et si l'effort que ce dernier fournit à un instant t est compatible avec le reste d'un itinéraire à parcourir.

Le document US 7,534,206 divulgue un dispositif portatif d'assistance à la gestion d'un effort physique fourni par un sujet selon le préambule de la revendication 1.

Il existe donc un besoin pour un dispositif capable non seulement de recueillir, enregistrer et afficher des paramètres physiologiques relatifs à un sujet, qu'il soit animal ou humain, mais aussi de les prévoir sur au moins une portion d'un trajet restant à parcourir sur un itinéraire prédéterminé, en particulier en fonction de la position du sujet sur cet itinéraire.

De plus, il existe un besoin pour un tel dispositif qui soit portatif afin qu'un sujet puisse l'emporter avec lui sur un itinéraire réel et disposer d'une assistance à la gestion de son effort en tous points de son parcours, en temps réel, et de façon autonome.

La présente invention vise donc de manière générale à résoudre le problème de la prévision d'un ou plusieurs paramètres physiologiques en fonction d'un trajet restant à parcourir par un sujet sur un itinéraire réel prédéterminé.

L'invention vise plus particulièrement à proposer un dispositif permettant de prévoir l'évolution d'un ou plusieurs paramètres physiologiques en fonction du trajet restant à parcourir par un sujet sur un itinéraire prédéterminé.

L'invention vise en particulier, dans ce cadre, à prévoir au moins le rythme cardiaque du sujet sur au moins un intervalle de temps ou de distance à partir d'au moins un point d'un trajet restant à parcourir.

L'invention vise aussi un dispositif qui soit portatif et autonome, afin de procurer une autonomie complète du sujet sur un itinéraire réel.

L'invention vise également à fournir un dispositif capable d'affiner des prévisions de paramètres d'effort en fonction de facteurs environnementaux tels que météorologiques notamment.

L'invention vise encore à proposer un dispositif capable de prévoir d'autres paramètres physiologiques du sujet tels que par exemple l'oxymétrie, la température, l'énergie consommée et l'énergie restante.

L'invention vise notamment à proposer un dispositif d'assistance à la gestion d'un effort physique pouvant être mis en oeuvre aussi bien pour un animal que pour un être humain.

L'invention vise aussi à fournir un dispositif d'assistance à la gestion d'un effort physique relatif à une rééducation, à un entraînement ou à une compétition sportive.

L'invention vise à fournir un dispositif d'assistance à la gestion d'un effort physique applicable à tous types d'efforts physiques, et en particulier dans tous types de sports.

Pour ce faire l'invention concerne un dispositif portatif d'assistance à la gestion d'un effort physique fourni par un sujet comprenant :

- au moins un capteur d'un paramètre physiologique, dit paramètre d'effort, évoluant en fonction de l'effort physique, ledit capteur étant apte à fournir des données numériques, dites données d'effort, représentatives dudit paramètre d'effort,
- au moins un dispositif, dit dispositif de positionnement, apte à déterminer sa propre position dans un référentiel topographique donné et à fournir des données numériques, dites données de positionnement, représentatives de cette position,
- au moins une interface de communication avec un utilisateur,
- au moins une mémoire dans laquelle sont susceptibles d'être enregistrées des données, dites données d'entrées, comprenant au moins :

  o des données de positionnement fournies par ledit dispositif de positionnement,
  o des données d'effort,
  o des données représentatives d'un itinéraire à parcourir dans ledit référentiel topographique par ledit sujet au cours de l'effort physique,

- au moins une unité de traitement de données, agencée pour :

  ▪ produire des données, dites données prévisionnelles, représentatives de l'évolution d'au moins un paramètre d'effort sur l'itinéraire restant à parcourir par le sujet, en fonction des données d'entrées, à partir d'équations et de tableaux de valeurs enregistrés dans au moins une mémoire du dispositif,
  ▪ comparer ces données prévisionnelles avec des valeurs prédéterminées enregistrées dans au moins une mémoire dudit dispositif d'assistance,
  ▪ produire des données représentatives d'un message dont le contenu est fonction du résultat de la comparaison des données prévisionnelles et des valeurs prédéterminées,
  ▪ transmettre lesdites données représentatives d'un message à l'interface en vue de la communication dudit message à un utilisateur.

L'invention est applicable à tout sujet faisant partie du règne animal au sens biologique du terme. C'est-à-dire que le sujet peut être un animal au sens commun du terme, ou un être humain.

Il est à noter cependant que l'utilisateur du dispositif selon l'invention peut être distinct du sujet. Dans le cas où le sujet est un cheval par exemple, l'utilisateur du dispositif peut être le cavalier. De même un sportif peut être le sujet et son entraîneur, l'utilisateur.

Un dispositif selon l'invention est au moins partiellement portatif, en particulier, au moins les capteurs d'efforts doivent être dimensionnés de telle manière à pouvoir être portés par le sujet.

La portabilité d'un tel dispositif trouve une utilité certaine lorsqu'il s'agit de l'utiliser sur un sujet ne disposant pas d'un véhicule, c'est-à-dire en particulier pour des sports ou activités physiques non-motorisées (marche, course à pied, cyclisme, natation, équitation, etc.). Un tel dispositif doit être portatif dans le sens où il est facilement porté par un sujet en train de faire un effort physique sans relation avec le fait de porter le dispositif. Un tel dispositif doit donc présenter un poids très inférieur au poids de l'individu (par exemple une centaine de grammes au maximum pour un être humain), et des dimensions relativement réduites par rapport au sujet (par exemple 100x60x30 mm pour un être humain). Idéalement, le dispositif est assez léger et petit pour être porté par un sujet pendant ses efforts physiques sans que le fait de porter le dispositif n'influe de façon notoire sur la performance physique du sujet pendant son effort. Par exemple, le dispositif peut prendre la forme d'un bracelet à porter au poignet pour un sujet humain.

Un dispositif selon l'invention peut être utilisé dans des situations d'effort physique diverses. Ainsi, l'invention peut s'appliquer à un dispositif destiné à assister la rééducation de sujets suite à une opération et/ou à un traumatisme. Il peut aussi être utilisé dans des situations sportives, à l'entraînement ou en compétition.

Par ailleurs, les activités dans lesquelles un dispositif selon l'invention est applicable sont très variées. Toutes les activités impliquant un effort physique, qu'il soit intense ou non, peuvent justifier l'utilisation d'un dispositif selon l'invention. Rien n'empêche par exemple de l'utiliser dans des épreuves sportives de type sport automobile afin de surveiller et prévoir un ou plusieurs paramètres physiologiques ou encore l'état physique et/ou de fatigue général d'un pilote. Rien n'empêche non plus d'appliquer l'invention dans des domaines où la gestion du stress du sujet est importante, par exemple dans des compétitions de vol à voile ou de parapente.

En particulier, un même dispositif selon l'invention n'est pas dédié à un unique type d'effort physique. Un tel dispositif est agencé pour pouvoir fournir des données prévisionnelles dans de nombreux types d'efforts physiques. Par exemple, un tel dispositif peut être utilisé régulièrement et indifféremment pour de la course à pied, du vélo, de la natation. Ainsi, par exemple, un sujet pratiquant le triathlon pourra utiliser le même dispositif tout au long de l'épreuve sportive, éventuellement en indiquant au dispositif un changement de mode d'effort physique grâce à une interface. Avantageusement, un profil d'utilisateur sera déjà connu du dispositif lorsque l'utilisateur abordera un nouveau type d'effort physique et les prévisions seront d'autant plus précises, sans apprentissage préalable du dispositif sur ce type d'effort.

Les données d'effort, d'itinéraire, prévisionnelles, de positionnement sont des données numériques représentatives de valeurs. Ainsi, par exemple, on utilise dans la suite le terme « valeurs prévisionnelles » ou « valeurs programmées » pour désigner les différents types de valeurs représentés par les « données prévisionnelles ».

De plus un dispositif selon l'invention comprend des capteurs physiologiques aptes à convertir un paramètre physiologique susceptible d'être modifié lors d'un effort physique, notamment en fonction de l'intensité de l'effort physique, en données numériques représentatives de la mesure réalisée. Par exemple et avantageusement, un tel capteur physiologique peut convertir le rythme cardiaque d'un sujet en données numériques. Il va de soi que différents types de capteurs basés sur différents types de technologies (audio, optique, thermique, etc.) peuvent être choisis en fonction de leurs avantages connus pour chacun des paramètres d'effort à mesurer.

Un dispositif de positionnement selon l'invention permet de fournir des données de positionnement en temps réel relatives à la position d'un dispositif selon l'invention, et donc à la position du sujet lorsqu'il est à proximité dudit dispositif. Cette position comporte avantageusement trois données de positionnement : latitude, longitude et altitude. Ces données sont relatives à l'environnement dans lequel se trouve le sujet : sur Terre ou sur un autre astre du système solaire, dans une grotte, etc.

Les données de géo-positionnement permettent notamment de calculer la dépense énergétique en fonction du parcours. Ainsi, le dénivelé donne une indication de l'énergie potentielle que le sujet devra fournir,

Un dispositif de positionnement selon l'invention peut utiliser différents types de technologies de localisation. Notamment, un dispositif de positionnement selon l'invention est apte à trianguler des données transmises par les satellites d'un système de positionnement mondial (GPS, Galileo, etc.). Il peut alternativement ou en combinaison déterminer sa position à partir d'un réseau d'antennes, par exemple de type téléphonie mobile (GSM, etc.). Enfin, un dispositif de positionnement peut, seul ou en combinaison avec les technologies précédemment décrites, déterminer sa position grâce au calcul des déplacements réalisés à partir d'une certaine position connue. Dans ce dernier cas, la connaissance des déplacements implique de mesurer les changements de trajectoire, par exemple grâce à un accéléromètre, la vitesse de déplacement du sujet et le temps écoulé entre chaque changement de trajectoire. D'autres moyens de localisation peuvent être envisagés selon l'invention, seuls ou en combinaison avec les moyens précédemment décrits.

Un dispositif de positionnement selon l'invention est relatif à un positionnement et est agencé pour fonctionner sur des parcours réel, c'est-à-dire que l'on peut trouver en extérieur (routes, chemin, itinéraires non-balisés, etc.). Un tel dispositif ne s'applique pas dans le cadre de parcours virtuels simulés sur des machines d'entraînement en intérieur.

Un dispositif de positionnement selon l'invention est apte à accéder et à lire des cartes, notamment sous forme de fichiers. Les cartes peuvent être enregistrées en mémoire ou téléchargées depuis un dispositif distant. Notamment les cartes peuvent être sous la forme d'un ou plusieurs fichiers séparés.

De plus, un dispositif de positionnement selon l'invention est agencé pour calculer un itinéraire entre une première position, notamment sa propre position, et une seconde position, par exemple fournie comme donnée d'entrée par un utilisateur du dispositif d'assistance. Un tel dispositif est agencé pour pouvoir calculer un itinéraire selon un ou plusieurs critères (temps de parcours, dénivelé total minimum, distance, etc.) à optimiser.

Un dispositif de positionnement selon l'invention est apte à au moins fournir une représentation altimétrique de l'itinéraire à parcourir. En effet, l'information de relief (pentes positives, pentes négatives, plateaux) est nécessaire à la prévision de paramètres physiologiques du sujet, au moins lorsqu'il évolue au sol.

En outre, un dispositif de positionnement selon l'invention est adapté pour mettre à jour un itinéraire en temps réel en fonction d'une position mesurée qui évolue.

En outre, avantageusement, un dispositif selon l'invention comporte une interface adaptée pour un utilisateur humain.

L'interface peut être réalisée selon plusieurs modalités de communication avec l'utilisateur humain : optique (par exemple un écran), acoustique (par exemple un dispositif d'alarme sonore), haptique (par exemple un dispositif apte à transmettre une sensation sur un doigt), une combinaison de ces moyens, ou autre. Ainsi une interface peut être un simple moyen de communication, avec ou sans fil, permettant au dispositif d'envoyer et/ou recevoir des informations d'un autre dispositif.

L'interface avec un utilisateur permet à l'utilisateur d'interagir avec le dispositif. Elle permet en particulier de régler le dispositif selon les différents modes de fonctionnement intégrés au dispositif.

Par exemple, avantageusement, un dispositif selon l'invention peut proposer à un utilisateur de choisir un sport et à un mode d'effort physique, par exemple un mode « rééducation », un mode « entraînement » et un mode « compétition ». Ces données d'entrée permettent audit dispositif de sélectionner les équations et/ou abaques à utiliser et les valeurs seuil (ou fourchettes) à imposer.

Une interface d'un dispositif selon l'invention permet aussi à l'utilisateur de programmer l'itinéraire que le sujet parcourra.

En outre, dans le cas d'un dispositif pouvant réaliser des prévisions relatives à plusieurs paramètres physiologiques, l'interface peut permettre à l'utilisateur de choisir quels paramètres physiologiques il souhaite que le dispositif prévoie et/ou affiche. Rien n'empêche de prévoir que l'utilisateur puisse aussi choisir différents modes de représentation des données prévisionnelles : graphique ou numérique, selon un échantillonnage fonction du temps ou de la distance (moyenne glissante, valeurs instantanées, etc.).

De plus un dispositif selon l'invention comporte des mémoires, dont la technologie est choisie en fonction des caractéristiques techniques attendues d'une telle mémoire (persistance, vitesse de lecture/écriture, capacité, etc.). De telles mémoires sont adaptées pour stocker des données de positionnement, des données d'effort relatives à des paramètres physiologiques, des données d'itinéraire dans le même référentiel topographique que les données de positionnement. Avantageusement et selon l'invention, de telles mémoires sont agencées pour aussi stocker d'autres types de données : formules et abaques utiles aux opérations menées par l'unité de traitement, et/ou autres.

Une unité de traitement de données selon l'invention comporte avantageusement au moins un processeur, et éventuellement des moyens de stockage de données internes (mémoire vive, mémoire dure, etc.). Une unité de traitement de données dans un dispositif selon l'invention est agencée pour pouvoir recevoir et traiter les données numériques issues du(des) capteur(s) physiologique(s), du dispositif de positionnement et du dispositif de calcul d'itinéraires et/ou récupérer ces données dans lesdites mémoires du dispositif et/ou ses propres mémoires internes.

En particulier, une unité de traitement selon l'invention est agencée pour suivre au moins les cinq étapes suivantes :

   a) accéder à des données d'entrée enregistrées dans au moins une mémoire parmi lesquelles:

      o des données de positionnement,
      o des données représentatives d'un paramètre d'effort à cette position,
      o des données représentatives d'un trajet restant à parcourir sur l'itinéraire à parcourir,

   b) appliquer à ces données des équations et/ou abaques, biologiques et/ou mécaniques, dépendantes du type d'effort et/ou du sujet, pour produire des données prévisionnelles représentatives de l'évolution future d'au moins un paramètre d'effort,
   c) comparer ces données prévisionnelles à des valeurs prédéterminées, notamment enregistrées dans une mémoire, lesdites valeurs prédéterminées ayant été saisies manuellement par un utilisateur ou calculées par l'unité de traitement de données,
   d) produire des données représentatives d'un message en fonction du résultat de l'étape c), notamment produire des données représentative d'un conseil ou d'une alarme à destination d'un utilisateur du dispositif si au moins un paramètre d'effort est au-delà ou en deçà des valeurs prédéterminées,
   e) transmettre lesdites données représentatives d'un message à ladite interface afin que le contenu informatif du message puisse être compris d'un utilisateur.

En particulier, l'étape a) peut être réalisée par l'accès à des données stockées en mémoire vive provenant directement respectivement du dispositif de positionnement, du capteur physiologique, et du dispositif de calcul d'itinéraire.

A partir des données d'entrées précédemment citées, l'unité de traitement de données est apte à déterminer des données secondaires, notamment grâce à un historique des données d'entrée. Par exemple, l'unité de traitement de données est apte à déterminer une vitesse instantanée et/ou une vitesse moyenne de déplacement du sujet à partir des données historiques et présentes de position.

L'algorithme mis en oeuvre par l'unité de traitement de données pour réaliser le calcul de la prévision du rythme cardiaque est de préférence de type itératif, par exemple un réseau neuronal. Les données instantanées sont comparées à chaque pas de temps à des valeurs cibles (objectifs) en relation avec des valeurs de référence (abaque) et avec des données de géo-positionnement et environnementales. Un tel algorithme peut par exemple comporter les étapes suivantes :

   - calcul d'une moyenne des paramètres physiologiques depuis le début du parcours à intervalles de temps ou de distance prédéterminés,
   - mesure d'écart entre les valeurs réelles mesurées et les valeurs programmées calculées par le système comme données de référence pour optimiser l'effort physique sur l'itinéraire à parcourir, à partir du profil du sujet, d'abaques et des données d'itinéraire,
   - adaptation des valeurs programmées (par exemple fréquence cardiaque, vitesse) sur le reste du circuit, en fonction des écarts entre les valeurs mesurées des paramètres physiologiques (fréquence cardiaque, SaO2, température, ...) et les valeurs cibles ou les seuils d'alerte.

Les équations et les abaques, biologiques et/ou mécaniques utilisés pour la prédiction de paramètres physiologiques peuvent être choisis en fonction du type d'effort (en particulier en fonction du sport), et en fonction du sujet. De tels abaques sont disponibles dans la littérature.

De plus, les constantes de certaines équations peuvent être adaptées après chaque effort physique pour mieux représenter le sujet. Il peut aussi être proposé à l'utilisateur d'entrer manuellement des caractéristiques spécifiques du sujet (poids, taille, âge, sexe, etc.) qui sont alors mémorisées par le dispositif.

Avantageusement, la prévision d'un paramètre d'effort est réalisée avec une précision minimale qui dépend du paramètre (par exemple de l'ordre de $\pm5$bpm pour la fréquence cardiaque, de $\pm2\%$ pour la saturation en oxygène SaO2, etc.).

En outre, en fonction des paramètres d'effort calculés, l'horizon de prévision, c'est-à-dire la durée/distance maximale pour laquelle il est utile de réaliser une prévision dudit paramètre, peut être différent. Ainsi, pour des paramètres d'effort dit de « court-terme », c'est-à-dire qui sont facilement et rapidement (de l'ordre de quelques minutes) modifiés par des changements d'allure, par des conditions environnementales changeantes, etc., l'horizon de prévision est relativement court : il suffit que le dispositif estime ce paramètre uniquement sur quelques unités de temps/distance. Par exemple, la fréquence cardiaque peut être estimée avec un horizon d'environ dix minutes. Au delà, les prévisions sont fortement dépendantes des autres paramètres. Par exemple, il est possible, pour un sujet dont la fréquence cardiaque est trop élevée, de la faire redescendre en quelques minutes, sans incidence sur sa capacité à remonter à une fréquence cardiaque élevée plus tard.

Au contraire, d'autres paramètres peuvent être considérés comme des paramètres à « long-terme ». Ce sont des paramètres qui peuvent être estimés jusqu'à la fin de l'effort physique car ils sont moins facilement modifiés. Par exemple, l'énergie consommée par un sujet ne peut pas être récupérée, ni en quelques minutes ni en quelques heures, tant que le sujet n'ingère pas d'aliment. L'horizon de prévision d'un paramètre de « long-terme » peut donc être étendu jusqu'à la fin de l'effort physique.

Ainsi, un dispositif selon l'invention calcule des valeurs, dites valeurs programmées, pour paramètres « court-terme » (Vitesse, Fréquence Cardiaque, ...) et « long terme » (ex. énergie emmagasinée, quantité d'eau, etc.) avant le début de l'effort, sur l'ensemble de l'itinéraire. Ces valeurs programmées correspondent à une optimisation de l'effort à fournir par le sujet le long de l'itinéraire en fonction notamment des caractéristiques du sujet, du mode d'effort physique, de la topologie de l'itinéraire, et éventuellement de paramètres environnementaux. De nombreux autres paramètres peuvent être pris en compte dans le calcul de ces valeurs programmées.

De plus, le dispositif calcule des valeurs prévisionnelles au fur et à mesure de l'avancement du sujet sur l'itinéraire, avec un horizon plus restreint (par exemple 10 minutes) pour les paramètres « court terme » (par exemple la fréquence cardiaque). Ces valeurs prévisionnelles correspondent à une estimation de l'évolution future des paramètres physiologiques faisant l'objet d'une prédiction, en fonction des valeurs mesurées présentes (et éventuellement passées), de la topologie de l'itinéraire à venir immédiatement, et éventuellement de paramètres environnementaux. De nombreux autres paramètres peuvent être pris en compte dans le calcul de ces valeurs prévisionnelles.

Les valeurs mesurées et les valeurs prévisionnelles sont comparées en temps réel aux valeurs programmées. Elles sont également comparées à des valeurs seuil (par exemple une valeur de fréquence cardiaque à ne jamais dépasser).

De plus, un dispositif selon l'invention est agencé pour réanalyser régulièrement l'ensemble des paramètres et produire à intervalles de temps réguliers de nouvelles valeurs programmées en fonction notamment des valeurs passées sur la portion d'itinéraire déjà parcourue. Cela permet notamment de réévaluer la performance optimale du sujet sur un trajet restant à parcourir en fonction de sous-performances ou de surperformances sur la portion d'itinéraire déjà parcourue.

Avantageusement et selon l'invention, un premier paramètre d'effort est la fréquence cardiaque et au moins un second paramètre d'effort est choisi parmi : la saturation en dioxygène du sang, la température corporelle, le taux de glucose dans le sang, la tension artérielle, la température corporelle, la fréquence respiratoire, la conductivité de la peau.

La fréquence cardiaque est un paramètre d'effort à la fois simple à mesurer et très représentatif de l'état physique d'un sujet. Il est donc essentiel de mesurer la fréquence cardiaque dans un dispositif selon l'invention.

Mais une analyse plus exhaustive et une prévision plus précise des performances et des ressources d'un sujet requièrent de disposer de mesures d'autres paramètres d'effort (ou paramètres physiologiques). En effet, la fréquence cardiaque n'est pas suffisante pour obtenir des informations fiables et complètes sur l'état de l'organisme du point de vue de son métabolisme instantané.

Ainsi, la saturation en dioxygène du sang, mesurée par exemple par un saturomètre ou oxymètre, est un paramètre très important et nécessaire pour qu'un dispositif selon l'invention soit apte à prévoir, par exemple, l'état de fatigue ou la quantité d'énergie qui sera utilisée par le sujet sur le trajet restant à parcourir. Bien qu'optionnelle, cette mesure est utile pour l'évaluation de l'état physique du sujet et pour le calcul de la consommation énergétique du sujet durant l'effort.

D'autres paramètres physiologiques peuvent remplacer ou s'ajouter à la mesure de la saturation en dioxygène du sang, en fonction de la nature et de l'exactitude attendues des données prévisionnelles fournies par un dispositif selon l'invention. Ainsi, un dispositif selon l'invention peut comporter des capteurs physiologiques destinés à convertir en données numériques des paramètres physiologiques tels que : le taux de glucose dans le sang, la tension artérielle, la température corporelle, la fréquence respiratoire, la conductivité de la peau (permettant notamment de mesurer la sudation et donc la perte en eau), ou autres.

Ces capteurs, dans un dispositif selon l'invention visent à assurer un suivi plus complet de l'état physique du sujet, et donc à affiner en précision des données prévisionnelles telles que rythme cardiaque, énergie consommée, réserves en eau, etc.

Un dispositif selon l'invention ne se restreint pas aux capteurs physiologiques cités et peut comporter des capteurs physiologiques secondaires aptes à mesurer d'autres paramètres physiologiques. Ces mesures peuvent être utiles à affiner le calcul de certaines données prévisionnelles. Ainsi, la saturation en dioxygène du sang peut aider à prévoir le rythme cardiaque futur. De même, la saturation en dioxygène permet d'évaluer l'état physique du sujet, l'efficacité de la production énergétique par l'organisme, et donc d'estimer le niveau et l'évolution de la performance globale au cours de l'effort physique (cf. par exemple « Physiologie du sport et de l'exercice : Adaptations physiologiques à l'exercice physique », Jack H. Wilmore, David L. Costill, trad. Arlette Delamarche, Paul Delamarche et Carole Groussard, De Boeck Université, 2006.)

Le calcul des données prévisionnelles peut prendre en compte séparément ou en interaction les différents paramètres mesurés. C'est-à-dire qu'en fonctions des équations et/ou abaques appliqués (qui dépendent notamment du type d'effort) aux paramètres mesurés, ces derniers peuvent être pris en considération l'un après l'autre pour parvenir aux données prévisionnelles, ou bien ils peuvent être pris en considération ensemble lorsqu'ils interfèrent entre eux.

De plus, avantageusement et selon l'invention, ladite unité de traitement de données est agencée pour produire au moins une donnée prévisionnelle représentative d'une dépense énergétique en fonction des données d'entrée et de données représentatives de l'évolution d'au moins un paramètre d'effort.

Ainsi, le rythme cardiaque est un paramètre d'effort fourni en entrée de l'unité de traitement de données. Cependant le rythme cardiaque peut aussi être une donnée prévisionnelle lorsqu'un dispositif selon l'invention est adapté pour produire des données prévisionnelles représentatives de l'évolution future du rythme cardiaque. Le rythme cardiaque prévisionnel calculé à partir au moins du rythme cardiaque présent (instantané) est avantageusement affiné en fonction d'au moins une partie du reste des données d'entrée (rythme cardiaque passé, position sur un itinéraire, topologie du trajet restant, etc.).

De la même manière des calculs prédictifs de la saturation en dioxygène du sang, de la tension artérielle, de la température corporelle, de la fréquence respiratoire, du stress, ou autres, peuvent être envisagés dans le cadre de l'invention.

De plus, avantageusement et selon l'invention, ladite unité de traitement de données est agencée pour produire au moins une donnée prévisionnelle représentative d'une dépense énergétique en fonction des données d'entrée et de données représentatives de l'évolution d'au moins un paramètre d'effort.

Un dispositif selon l'invention ne se restreint pas à la prévision de paramètres physiologiques mesurables. En effet, la mesure d'un ensemble de paramètres physiologiques peut permettre de prévoir un ou plusieurs autre(s) paramètre(s) relatif(s) à l'état physique (ou physiologique) du sujet qui n'est(ne sont) pas mesurable(s) : par exemple, la fatigue ou la réserve en énergie d'un sujet qui peuvent être représentées et/ou calculées à partir de différents paramètres physiologiques et/ou de leur évolution dans le temps, et donc à partir de leurs valeurs présente et passées.

Certains paramètres physiologiques parmi lesquels : le rythme cardiaque, la glycémie, la saturation en dioxygène du sang, la tension artérielle, la température corporelle, la fréquence respiratoire, la conductivité de la peau, et autres, peuvent permettre, seul ou en combinaison(s) de calculer et de prévoir un niveau de fatigue, d'énergie consommée ou de réserves en énergie restante d'un sujet et de les comparer à un trajet restant à parcourir.

En général, dans un dispositif selon l'invention, un utilisateur doit apporter des données d'entrée telle que, par exemple la quantité d'énergie du sujet au départ de l'effort physique.

Par ailleurs, avantageusement et selon l'invention, l'unité de traitement de données est agencée pour comparer chaque paramètre d'effort à au moins une valeur, dite valeur seuil, prédéterminée et propre audit paramètre d'effort.

En particulier, un paramètre d'effort mesuré en temps réel est en permanence comparé à une valeur seuil (et/ou à une valeur plafond). De même, un paramètre d'effort calculé (ou donnée prévisionnelle) est aussi comparé à une valeur seuil afin de prévenir un éventuel dépassement futur de cette valeur seuil.

Avantageusement, pour les paramètres dits de « court-terme », tels que la fréquence cardiaque par exemple, qui évoluent rapidement, les données présentes et prévisionnelles (à court terme donc) sont comparées auxdites valeurs seuil. En ce qui concerne des paramètres dits de « long terme », tels que l'énergie disponible par exemple, la valeur actuelle est comparée à la prévision effectuée en début d'effort physique pour l'ensemble de l'itinéraire. Par exemple il peut être considéré comme avantageux de vérifier que l'énergie déjà consommée n'est pas supérieure à la prévision qui avait été effectuée en début de parcours pour ce point de l'itinéraire, car, si tel était le cas, cela signifierait que le sujet va devoir ralentir ou peut-être qu'il ne disposera pas de suffisamment d'énergie pour parvenir à sa destination.

Un dispositif selon l'invention est ainsi avantageux pour prévenir les accidents (cardio-vasculaires, hypoxie, hypoglycémie, etc.). Un tel dispositif est donc très avantageux pour les sujets en phase de rééducation. Un tel dispositif est capable de prévenir les risques avant que ceux-ci ne se manifestent.

Par ailleurs, avantageusement et selon l'invention, un dispositif d'assistance de gestion de l'effort physique comporte des moyens d'alarme activés par au moins une donnée prévisionnelle d'au moins un paramètre d'effort en dehors d'un intervalle de valeurs prédéterminée.

Rien n'empêche, dans un dispositif selon l'invention, que l'intervalle de valeurs pour un certain paramètre d'effort soit calculée par le dispositif à partir de données enregistrées. Ce calcul peut être effectué avant une séquence d'effort physique, après celle-ci, ou bien peut être actualisée au fur et à mesure en fonction des données collectées sur le sujet et permettant d'affiner le(s) modèle(s) biologique(s), mécanique(s) et/ou physiologique(s) pour ce sujet. Cependant, rien n'empêche non plus que cet intervalle soit enregistré par un utilisateur ou lors de la fabrication/programmation d'usine du dispositif. Avantageusement un intervalle standard est enregistré lors de la fabrication et il est affiné après chaque effort physique.

Un dispositif selon l'invention compare à intervalles réguliers la prévision d'un paramètre d'effort du sujet à cet intervalle afin de prévenir tout risque. Par exemple, dans le cas du calcul prévisionnel d'un taux de glycémie, le résultat de cette prévision doit se tenir dans un intervalle, et si le taux est inférieur à la valeur minimale de l'intervalle, ou supérieur à la valeur maximale de l'intervalle, le sujet encoure des risques qu'un dispositif selon l'invention est apte à anticiper et à signaler à un utilisateur grâce auxdits moyens d'alarme.

Les moyens d'alarmes d'un dispositif selon l'invention peuvent être de multiples natures, et en particulier peuvent utiliser une interface du dispositif. Ainsi une alarme peut être :

- sonore, ce peut être un son ou un message audio par exemple,
- optique, ce peut être un affichage graphique, de couleur, numérique, ou tout autre moyen de représentation visuel jugé approprié,
- ou haptique, ce peut être une sensation, une vibration ou une sensation de piqûre sur le doigt par exemple.

Une alarme selon l'invention peut être réalisée de différentes manières mais aussi et surtout dans différents objectifs. En effet, si la prévision indique qu'un paramètre d'effort va sortir d'un intervalle, cette information peut être utilisée de différentes manières. Un dispositif selon l'invention peut par exemple émettre une alarme si le rythme cardiaque prévu est trop lent, mais il peut aussi émettre une indication de basse performance permettant d'optimiser les performances sportives du sujet. De même une alarme émise parce qu'une prévision est au-dessus d'un intervalle peut l'être parce que le sujet est en danger, ou simplement parce que cela risque de nuire à sa performance globale sur l'ensemble du trajet.

Un dispositif selon l'invention peut donc être utilisé tant à des fins d'entraînement et d'amélioration des performances, par exemple dans l'objectif d'améliorer ses performances sportive, qu'à des fins de prévention, par exemple dans le cadre d'une rééducation postopératoire.

Ainsi, une alarme selon l'invention s'étend à des dispositifs d'assistance aptes à donner des conseils à l'utilisateur et/ou au sujet tels qu'accélérer, ralentir, conserver sa vitesse actuelle, afin de conserver les paramètres d'effort dans leur limites, éventuellement en optimisant la performance physique.

Avantageusement, un dispositif selon l'invention, comporte au moins un capteur environnemental apte à fournir des données, dites données environnementales, relatives à l'environnement du dispositif.

Conformément à l'invention, un tel capteur environnemental peut être intégré au dispositif ou être à proximité et en communication avec ledit dispositif.

Un capteur environnemental selon l'invention peut par exemple mesurer l'humidité ambiante, la température ambiante, le vent, la pression, le taux d'un gaz particulier de l'atmosphère dans lequel le sujet évolue. De nombreux autres paramètres relatifs à l'environnement du sujet peuvent avantageusement être mesurés par un dispositif selon l'invention.

L'invention peut également être équipée d'un modèle d'atmosphère donnant les taux conventionnels de gaz dans l'atmosphère, ainsi que la pression et la température de celle-ci en fonction de l'altitude.

Avantageusement et selon l'invention, l'unité de traitement de données est agencée pour produire des données prévisionnelles au moins partiellement dépendantes desdites données environnementales.

Il est avantageux que l'unité de traitement des données soit apte à utiliser des données fournies par un ou plusieurs capteur(s) environnemental(aux) pour le calcul d'une prévision d'évolution d'un paramètre d'effort.

En effet, l'influence de l'environnement sur le sujet peut avoir un impact très important sur certains des paramètres physiologiques futurs du sujet. Aussi la prise en compte de certains facteurs environnementaux peut être déterminante pour une prévision fiable de ces paramètres. Par exemple, la fréquence respiratoire et la fréquence cardiaques dépendent de l'altitude, et compensent la hausse ou la baisse de pression et pour maintenir une saturation en oxygène stable ou cohérente avec le niveau d'effort fourni.

De même, les dépenses énergétiques et en eau sont fortement influencées par la température et l'hygrométrie environnantes. Ainsi, la mesure de la température extérieure permet d'estimer la déperdition en eau (température élevée) ou la surconsommation énergétique pour maintenir la température corporelle (basse température). Une perte de 2% du poids du corps pendant l'activité physique occasionnée par la transpiration est une déshydratation modérée mais qui peut nuire de manière significative aux performances.

Par ailleurs, la dépense énergétique du sujet sera aussi plus importante face au vent que dos au vent. Le vent peut par exemple être pris en compte dans la dépense énergétique (loi de résistance en $V^3$, V étant la vitesse). Le vent peut être une variable entrée manuellement par un utilisateur (soit une seule valeur soit une valeur correspondant à chaque point ou portion de l'itinéraire à parcourir), mesurée et/ou calculée par le système de positionnement (activités aériennes par exemple, par mesure de la dérive), ou encore téléchargée avant ou au fur et à mesure du déroulement de l'effort via des moyens de communication.

La liste envisagée de capteurs relatifs à l'environnement du sujet n'est pas exhaustive et tout autre capteur jugé nécessaire pour la prévision efficace d'un paramètre d'effort du sujet peut être intégré ou connecté à un dispositif selon l'invention.

Les capteurs environnementaux utilisés pour chaque paramètre peuvent être de tous types et sont choisis en fonction de leurs avantages connus et des caractéristiques attendues d'un dispositif selon l'invention.

Des capteurs additionnels peuvent être envisagés pour améliorer les prévisions du dispositif. Par exemple, un accéléromètre peut être embarqué à bord d'un véhicule piloté par le sujet, ou sur le sujet lui-même. Un accéléromètre, dans certaines situations peut permettre d'affiner, voire de remplacer les données de localisation par GPS. Les signaux délivrés par un accéléromètre peuvent aussi servir à évaluer le niveau de fatigue du sujet : un sujet ayant subit des accélérations répétées et/ou intenses dans un intervalle de temps réduit risque d'être plus fatigué.

Par ailleurs, avantageusement et selon l'invention, un dispositif d'assistance à la gestion d'un effort est adapté pour, après chaque effort, modifier et enregistrer dans une mémoire au moins un profil d'utilisateur constitué d'au moins une valeur utile à au moins une équation utilisée par l'unité de traitement de données pour produire des données prévisionnelles.

Ainsi, certaines données relatives au sujet peuvent être affinées dans le temps grâce à l'accumulation et à l'analyse de données enregistrées par le dispositif au cours de chaque effort physique.

En particulier après chaque session d'effort, un tel dispositif peut ajuster les constantes utiles à la réalisation de prévisions en fonction des performances réellement mesurées sur l'itinéraire. De même un tel dispositif peut ajuster les seuils limites (et/ou intervalles) d'alarme de certains paramètres pour permettre d'obtenir un dispositif plus adapté à un sujet en particulier.

Un dispositif selon l'invention est en particulier agencé pour pouvoir enregistrer et faire évoluer plusieurs profils d'utilisateurs, un profil d'utilisateur devant être sélectionné avant chaque effort.

Un dispositif selon l'invention peut être capable d'affiner un ou plusieurs profils d'utilisateurs enregistrés. Avantageusement, le dispositif affine les constantes des formules utilisées et/ou les valeurs seuils (ou intervalles) de déclenchement d'alarme après chaque effort physique.

Ainsi les valeurs de caractéristiques propres au sujet enregistrées lors d'itinéraires déjà parcourus permettent d'affiner les caractéristiques définies par défaut lors de la fabrication, ou des caractéristiques déjà individualisées (données d'entrées fournies par l'utilisateur ou calcul déjà effectué par le dispositif). Il s'agit donc d'améliorer et d'adapter en permanence le profil du sujet utilisé pour définir les valeurs prévisionnelles et les valeurs programmées. Ainsi, avantageusement, un utilisateur peut mettre à jour des paramètres du profil d'un sujet à l'aide de parcours types (épreuves et/ou entraînements spécifiques) afin de refléter une réelle prise en compte de l'évolution des performances du sujet.

De plus, un dispositif selon l'invention est avantageusement agencé pour produire des données prévisionnelles pour au moins deux types d'effort physique distincts.

Ainsi, un utilisateur peut, par exemple, modifier en cours d'itinéraire le type d'effort physique fourni, comme lors d'un triathlon. L'utilisateur peut aussi, par exemple, après avoir parcouru un premier itinéraire selon un premier type d'effort physique, reprogrammer un second itinéraire à parcourir, dans un second type d'effort différent du premier, et sans nécessiter de reconfigurer, ou de connecter le dispositif avec un autre dispositif externe tel qu'un ordinateur personnel.

Un tel dispositif doit donc comporter en mémoire des équations et/ou abaques de valeurs relatives à au moins deux types d'effort différents. L'utilisateur est alors à même de sélectionner, grâce à au moins une interface du dispositif, un mode d'effort physique.

De nouveaux types d'effort physique peuvent être ajoutés au dispositif en chargeant dans ses mémoires des fichiers relatifs au nouveau type d'effort, notamment de nouvelles équations (ou constantes d'équations) et/ou de nouveaux abaques. Avantageusement, même lorsque le profil d'un sujet est enregistré pour certains types d'effort, le dispositif selon l'invention utilise ce profil pour améliorer les prévisions relatives à un nouveau type d'effort. L'apprentissage du dispositif est donc croisé entre différents types d'effort physique. Cela est rendu possible par le fait que les grandes constantes physiologiques sont attachées au sujet, et non au type d'activité (fréquence cardiaque minimale et maximale, saturation en dioxygène maximale, masse, taille, etc.).

En outre, un dispositif selon l'invention comprend avantageusement au moins un moyen de communication.

Un tel moyen de communication peut aussi bien être filaire ou sans fil. Un moyen de communication filaire peut être utile, notamment pour relier le dispositif à un second dispositif (qui peut être quelconque, ou être un second dispositif selon l'invention) et charger des données vers ou depuis le second dispositif.

Ainsi, un dispositif selon l'invention peut avantageusement être connecté à un ordinateur personnel pour un traitement avancé des données enregistrées par un dispositif d'assistance à l'effort physique et/ou charger des données d'entrées sur ce dernier. Par exemple, rien n'empêche, selon l'invention, qu'un utilisateur puisse charger sur un dispositif selon l'invention, des cartes, des itinéraires, des équations et/ou abaques relatifs à un type d'effort, des profils d'utilisateur, des données prévisionnelles relatives à des paramètres d'effort sur un itinéraire donné, etc. Avantageusement, un tel dispositif est apte à acquérir des données environnementales telles que des prévisions météorologiques à partir d'un dispositif externe tel qu'un ordinateur personnel, notamment connecté à l'Internet.

Un moyen de communication sans fil peut être utilisé dans le même objectif mais présente en plus d'autres avantages. Par exemple, le dispositif de positionnement peut recevoir des données lui permettant de calculer sa position ; il dispose dans ce cas d'un récepteur sans fil. Cependant, un dispositif de positionnement selon l'invention peut aussi s'appuyer sur une information de positionnement entièrement calculée et fournie par un dispositif externe avec lequel elle est reliée par une communication sans fil. De même des informations relatives à la météorologie au point où se trouve le sujet peuvent être communiquées par liaison sans fil et en direct, ce qui peut permettre à l'unité de traitement d'affiner ses prévisions de paramètres d'effort ; par exemple, des données relatives à la vitesse et à la direction du vent, à la température, à l'hygrométrie, etc.

De plus rien n'empêche que ces moyens de communication servent à émettre un appel d'urgence lorsque qu'un paramètre d'effort dépasse une valeur prédéterminée, notamment dans le cadre de la rééducation d'un patient. Ce mode de réalisation peut aussi être utile, par exemple, à des personnes dont on sait qu'elles présentent un risque de santé particulier : par exemple une fragilité cardiovasculaire.

Enfin, de tels moyens de communication peuvent permettre de réaliser un dispositif réparti sur et/ou à proximité du sujet.

De plus, avantageusement et selon l'invention, un dispositif d'assistance à la gestion de l'effort comporte une pluralité d'unités réparties spatialement et reliées en réseau.

Ainsi, rien n'empêche par ailleurs de prévoir une unité de traitement des données apte à prendre en compte, non seulement les données des capteurs intégrés physiquement au dispositif, mais aussi des données issues de capteurs extérieurs. L'unité de traitement des données est notamment apte à traiter des données reçues depuis un capteur électriquement branché au dispositif ou en communication sans fil avec celui-ci.

Cette fonctionnalité permet d'étendre le nombre de paramètres physiologiques surveillés sans augmenter l'encombrement d'un dispositif selon l'invention. Cela permet aussi une grande modularité d'un tel dispositif : seuls les capteurs utiles à un type d'effort en particulier sont installés sur le sujet.

De tels capteurs font partie du dispositif portatif selon l'invention. Ainsi un dispositif selon l'invention peut se présenter sous la forme d'un nuage de capteurs et/ou dispositifs et/ou d'unités en réseau local corporel (Body Area Network (BAN)) sur le sujet et/ou à proximité du sujet.

En particulier, un dispositif selon l'invention peut présenter une unité sous la forme d'un bracelet portatif intégrant notamment le dispositif de positionnement, l'unité de traitement des données, une interface avec l'utilisateur et éventuellement un ou plusieurs capteur(s) physiologique(s) et/ou environnemental(aux). Le(s) capteur(s) physiologique(s) et/ou environnemental(aux) peu(ven)t être réparti(s) sur ou à proximité du sujet. Par exemple on mesure avantageusement la fréquence respiratoire d'un sujet grâce à un capteur disposé autour de la poitrine du sujet, ce qui n'est pas forcément compatible avec la facilité d'utilisation et l'interface visuelle disposée dans un bracelet. Les deux éléments d'un tel dispositif selon l'invention sont donc reliés par une liaison filaire ou sans fil.

L'invention s'étend à un dispositif d'assistance à l'effort caractérisé en combinaison par tout ou partie des caractéristiques mentionnées ci-dessus ou ci-après.

D'autres caractéristiques, buts et avantages de l'invention ressortiront de la description détaillée qui suit, en référence aux dessins qui en représentent à titre d'exemple non limitatif un mode de réalisation préférentiel. Sur ces dessins :

- la figure 1 est une représentation schématique extérieure d'un bracelet d'un dispositif portatif selon l'invention,

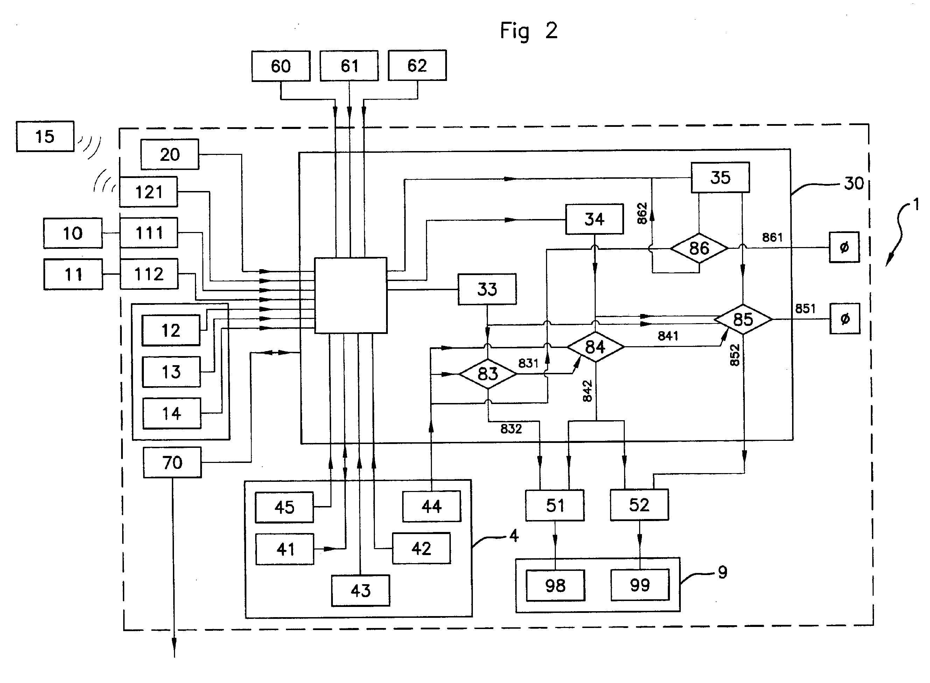

- la figure 2 est une représentation schématique du mode de réalisation d'un dispositif selon l'invention, illustrant les relations d'entrées/sorties entres certains éléments constitutifs du dispositif selon l'invention,
- la figure 3 est une représentation schématique illustrant des exemples de valeurs programmées de vitesse, de fréquence cardiaque et d'énergie restante en fonction de la topologie d'un itinéraire, avant le début d'un effort physique de type course à pied sur cet itinéraire, calculées par un dispositif selon l'invention,
- la figure 4 est une représentation schématique des mesures passées instantanées de paramètres d'effort sur une première portion d'un itinéraire, et de valeurs prévisionnelles et programmées pour ces paramètres d'efforts calculées pour un dispositif selon l'invention, pendant une course à pied.

Dans un mode particulier de réalisation décrit ci-après et représenté sur les figures, un dispositif selon l'invention d'assistance à la gestion d'un effort physique est adapté pour que l'utilisateur et le sujet soit un même être humain.

Un tel dispositif présente une partie sous la forme d'un bracelet pouvant avantageusement être porté au poignet, au bras ou à la cheville.

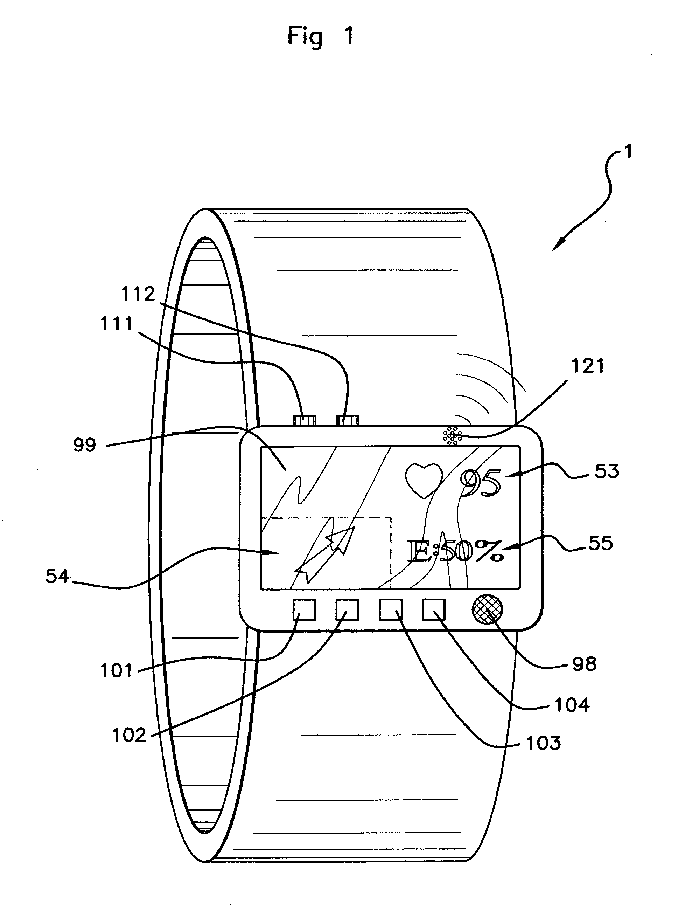

Le bracelet présente, vu de l'extérieur, un écran 99 et un haut-parleur 98, et des boutons 101 à 104 (qui pourraient avantageusement être remplacés ou complétés par une interface tactile) pour interagir avec l'utilisateur. Un tel dispositif peut, par exemple, afficher à l'écran 99, une valeur 33 mesurée du rythme cardiaque (dans la figure 1 : 95 battements par minute), une valeur 55 calculée du pourcentage d'énergie disponible au départ déjà consommée (dans la figure 1, 50% d'énergie consommée), et un indicateur relatif à un message 54 de conseil (sur la figure 1, un symbole fléché orienté vers le haut pour indiquer à l'utilisateur d'accélérer).

En outre ce bracelet présente des ports 111, 112 de connexion filaires et un port 121 de connexion sans fil pour pouvoir relier des capteurs ou des dispositifs ou des unités d'un dispositif portatif d'assistance à la gestion d'un effort selon l'invention au bracelet, de façon à former un dispositif portatif répartis sous la forme d'un réseau sur le corps (ou à proximité) du sujet.

Par ailleurs, un tel bracelet d'un dispositif portatif selon l'invention comprend :

- une unité 20 de réception de signaux GPS (Global Positioning System) et de calcul d'une position en latitude, longitude et altitude,
- une unité 30 de traitement de données,
- au moins une mémoire 4.

Le dispositif portatif selon l'invention comprend aussi au moins un capteur 10 de fréquence cardiaque, un capteur 11 de saturation en dioxygène du sang, et un capteur 12 de température corporelle qui peuvent être disposés dans le bracelet ou ailleurs sur le corps du sujet et en communication avec ledit bracelet. Par exemple, le capteur 10 de fréquence cardiaque peut être disposé sur la poitrine du sujet et relié au bracelet par l'intermédiaire du port filaire 111 ; le capteur 11 de saturation en dioxygène du sang sur un doigt du sujet et relié au bracelet par l'intermédiaire du port filaire 112 ; et le capteur 12 de température corporelle dans le bracelet portatif, du côté du bracelet qui est destiné à être en contact avec la peau du sujet.

Un dispositif 1 selon l'invention peut aussi comporter un capteur 15 de fréquence respiratoire, extérieur au bracelet, par exemple adapté à être disposé autour de la poitrine du sujet, et adapté pour pouvoir communiquer sans fil avec le bracelet via le port 121 de connexion sans fil.

Dans ce mode de réalisation de l'invention, le dispositif d'assistance à l'effort comporte aussi des capteurs environnementaux : un capteur 13 de température extérieure et un capteur 14 de pression atmosphérique.

Les données telles qu'itinéraire 60 (coordonnées du point de départ et coordonnées du point d'arrivée), énergie 61 disponible avant l'effort et type 62 d'effort physique sont entrées manuellement par un utilisateur avant le début de l'effort. L'unité de traitement de données est adaptée pour stocker ces données d'entrées au moins pendant la durée de l'effort dans une mémoire 41.

De plus, les données reçues des capteurs physiologiques 10, 11, 12 et environnementaux 13, 14, ainsi que les données de positionnement sont communiquées en en temps réel à l'unité 30 de traitement de données.

Ainsi, l'unité 30 de traitement de données récupère dans une ou plusieurs mémoire(s) 42, 43 les équations et abaques à appliquer aux données reçues en direct en fonction du type d'effort. A partir de ces dernières, l'unité de traitement est apte à fournir des données prévisionnelles sous la forme d'objectifs (valeurs programmées) relatifs à l'ensemble de l'itinéraire restant à parcourir, et des données prévisionnelles sous la forme de prévision (données prévisionnelles) à court-terme.

L'unité de traitement de données réalise à intervalles de temps réguliers une comparaison 83 entre les valeurs des données physiologiques mesurées 33 par les capteurs 10, 11, 12 physiologiques, et des valeurs limites (pouvant former une(des) fourchette(s)) auxquelles elle accède dans une mémoire 44. Si une valeur mesurée 33 excède ou est inférieure à une valeur limite (lien 832), un message 51 d'alarme est émis par l'unité de traitement de données pour être transmis à l'utilisateur via au moins une interface 9, par exemple, par l'intermédiaire d'un haut-parleur 98.

Si aucune valeur mesurée 33 n'excède ou est inférieure à une valeur 44 limite (lien 831), l'unité de traitement de donnée réalise une comparaison 84 entre les valeurs prévisionnelles 34 et les mêmes valeurs 44 limites.

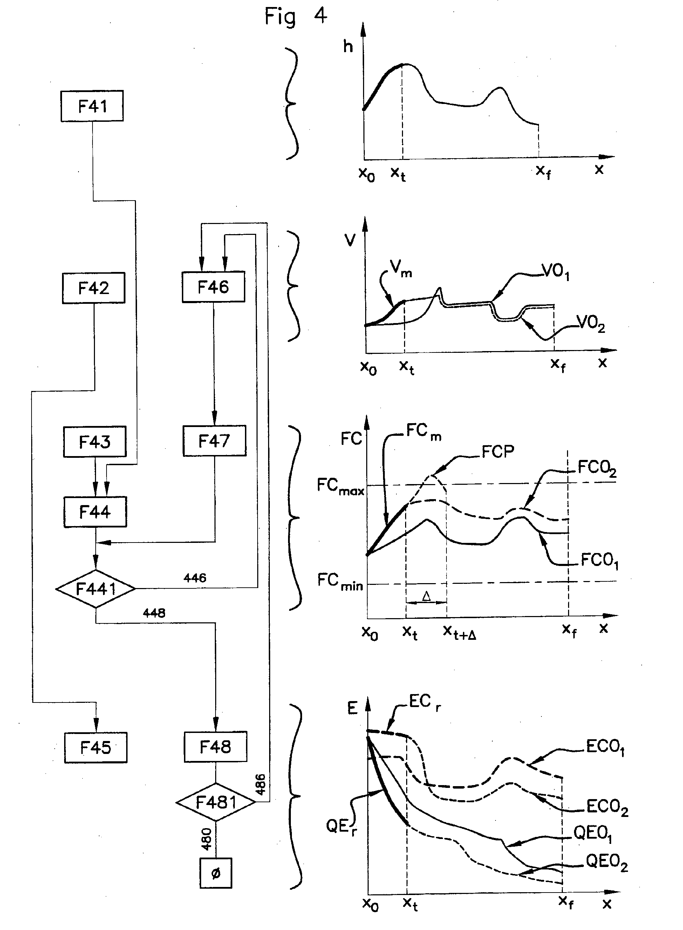

Si au moins une valeur 34 prévisionnelle excède ou est inférieure à une valeur 44 limite (lien 842), par exemple la fréquence cardiaque prévisionnelle FCP de la Figure 4, un message 51 d'alarme est émis et transmis à un utilisateur via au moins l'une des interfaces 98, 99.

Si l'ensemble des valeurs 34 prévisionnelles sont comprises dans les fourchettes de valeurs 44 limites (lien 841), l'unité de traitement de donnée réalise une comparaison 85 entre valeurs 33 mesurées et valeurs 35 programmées et une comparaison 85 entre valeurs 34 prévisionnelles et les mêmes valeurs 35 programmées. Si le résultat de la comparaison 85 indique que les valeurs 33 mesurées et les valeurs 34 prévisionnelles correspondent à un pourcentage d'erreur autour des valeurs 35 programmées (lien 851), l'unité 30 de traitement des données continue sa surveillance passive. Si les valeurs 33 mesurées et/ou 34 prévisionnelles sont en-dessous ou au-delà des valeurs 35 programmées (lien 852), l'unité 30 de traitement des données émet un message 52 de conseil qui est transmis à l'utilisateur par l'intermédiaire, par exemple, d'un écran 99, se traduisant pour un utilisateur par une indication visuelle 54.

Par ailleurs, avant le début de l'effort physique, une fois les valeurs programmées calculées, une comparaison 86 entre les valeurs programmées de fréquence cardiaque sur l'ensemble de l'itinéraire et une fourchette constituée d'une valeur minimale $FC_{min}$ et d'une valeur maximale $FC_{Max}$ (valeurs 44 limites) de la fréquence cardiaque du sujet, est ensuite effectuée. Les valeurs 44 limites formant une fourchette sont enregistrées dans une mémoire 4 du dispositif 1. Elles peuvent être entrées manuellement par un utilisateur, ou calculées en fonction d'autres paramètres fournis ou non par l'utilisateur. Par exemple, $FC_{min}$ et $FC_{Max}$ peuvent être calculées à partir de la taille, de l'âge et du poids de l'individu.

Si le résultat de la comparaison 86 indique qu'au moins une valeur des valeurs 35 programmées de la fréquence cardiaque est au-dessous de $FC_{min}$ ou au-delà de $FC_{Max}$ (lien 862), les valeurs programmées sont recalculées jusqu'à ce qu'elles satisfassent à ce critère. Si le résultat de la comparaison 86 indique que les valeurs 35 programmées de la fréquence cardiaque sont dans les limites de la fourchette [$FC_{min}$; $FC_{Max}$] (lien 861), le dispositif valide et conserve ces valeurs 35 programmées.

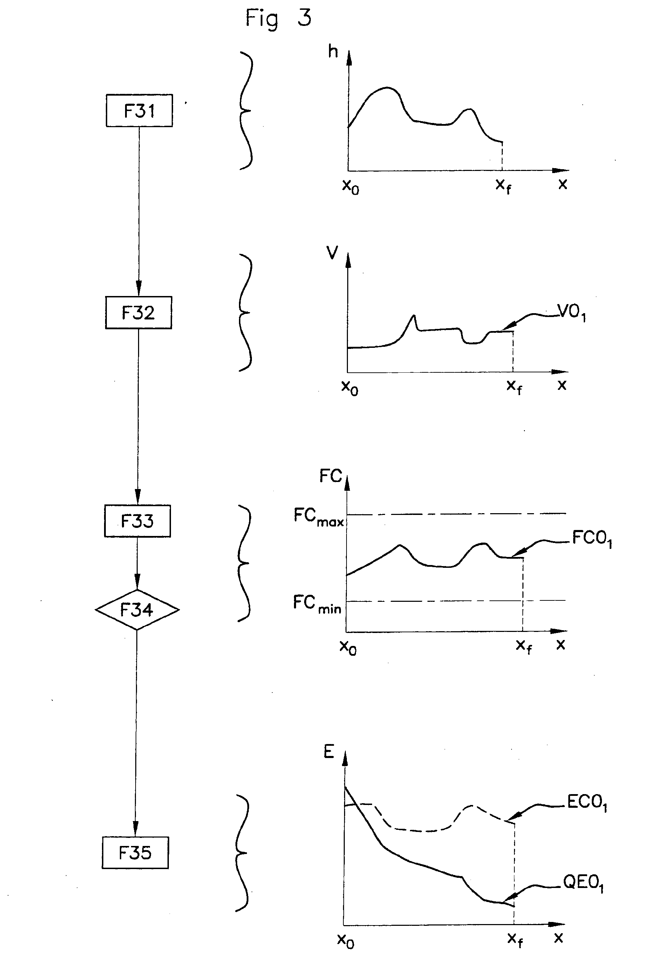

Dans l'exemple présenté en figures 3 et 4, le type d'effort physique est une course à pied dans laquelle le sujet est un être humain qui est aussi l'utilisateur du dispositif.

La figure 3 présente les valeurs programmées pour l'ensemble de l'itinéraire, calculées avant le début de la course, qui correspondent à des objectifs de performance. Dans les figures 3 et 4, h représente l'altitude en chaque point x de l'itinéraire entre le point de départ $X_0$ et le point d'arrivée $X_f$, V représente la vitesse en fonction des mêmes abscisses, FC la fréquence cardiaque et E l'énergie.

En particulier, sur la figure 3, $VO_1$ représente les valeurs programmées de la vitesse du sujet en chaque point x de l'itinéraire. $FCO_1$ représente les valeurs programmées de la fréquence cardiaque du sujet en chaque point x de l'itinéraire. $ECO_1$ représente les valeurs programmées de l'énergie consommée instantanée en chaque point de l'itinéraire et $QEO_1$ les valeurs programmées de la quantité d'énergie restante au sujet en chaque point x de l'itinéraire.

A l'étape F31, l'utilisateur fournit un itinéraire 60 à parcourir comme donnée d'entrée, soit en entrant un point de destination à partir de sa position actuelle, soit entre deux positions $X_0$ et $X_f$. Grâce aux cartes 45 préenregistrées dans une ou plusieurs mémoire(s) 4, le dispositif d'assistance est agencé pour réaliser une représentation topographique de l'itinéraire.

A partir de cette représentation topographique, à l'étape F32, le dispositif met en oeuvre le processus au cours duquel il produit des valeurs 35 programmées, c'est-à-dire sur l'ensemble de l'itinéraire, de la vitesse $VO_1$ du sujet en chaque point de l'itinéraire. Tout point x de l'itinéraire correspond à un instant t de la course une fois les valeurs programmées de la vitesse $VO_1$ calculées.

De la même manière, à l'étape F33, le dispositif produit des valeurs 35 programmées de la fréquence cardiaque $FCO_1$ du sujet. Le dispositif peut réaliser cette prévision de fréquence cardiaque $FCO_1$ à partir de la vitesse $VO_1$, et éventuellement des paramètres environnementaux (vent, température, ...). Cette prévision $FCO_1$ peut notamment être réalisée à partir d'abaque(s) 43 (la/les abaque(s) 43 peut(peuvent) être personnalisé(s) à un sujet particulier, lors d'un test à l'effort par exemple).

A l'étape F34, le dispositif effectue une comparaison 86 ($FC_{min} \leq FC \leq FC_{Max}$) entre valeurs 35 programmées de la fréquence cardiaque et les valeurs 44 limites $FC_{min}$ et $FC_{Max}$. Si au moins l'une des valeurs 35 programmées n'est pas dans cette fourchette, le profil $FCO_1$ est ajusté, c'est-à-dire que les valeurs programmées de la fréquence cardiaque sont modifiées jusqu'à ce qu'elles satisfassent à ce critère.

Le dispositif réalise ensuite une prévision d'énergie consommée, et donc une prévision d'énergie consommable restante à condition qu'un utilisateur ait fourni l'énergie consommable emmagasinée par le sujet en donnée d'entrée 61. L'énergie consommable 61 est constituée de l'énergie qui va être disponible pour l'effort physique envisagé sur l'itinéraire envisagé ; c'est-à-dire généralement les sucres rapides et les sucres lents, parfois certaines graisses.

A l'étape F35 le dispositif réalise une prévision à long-terme (calcul de valeurs 35 programmées), de la consommation d'énergie $ECO_1$ du sujet (et donc de la quantité d'énergie restante $QEO_1$). Par exemple, une équation permettant de réaliser cette prévision est (équation (1)):

$$\int_{x=X_0}^{x=X_f} \left(\frac{1}{2}mV(x)^2 + mg.d_h(x)\right).dx = E\left(\mathbf{X_0} \rightarrow \mathbf{X_f}\right)$$

Où m est la masse du sujet, g l'accélération de la pesanteur, V(x) la vitesse du sujet en valeur absolue en un point x de l'itinéraire, dh(x) le dénivelé parcouru au point x de l'itinéraire, et $E(X_0 \rightarrow X_f)$ l'énergie totale dépensée par le sujet sur l'ensemble de l'itinéraire (de $X_0$ à $X_f$). $E(X_0 \rightarrow X_f)$ est la valeur programmée de l'énergie consommée au point $X_f$, soit $E(X_0 \rightarrow X_f) = ECO_1(X_f)$.

Idéalement, l'énergie restante du sujet en tout point de l'itinéraire est très proche de la quantité d'énergie prévue à ce point. En particulier, en fin d'effort physique, un utilisateur peut viser une quantité d'énergie restante du sujet proche de zéro, ce qui signifie qu'il a utilisé le maximum de son potentiel énergétique sur cet itinéraire au court de l'effort physique.

Si l'énergie consommable restante du sujet est inférieure ou égale à zéro avant la fin de l'itinéraire, cela signifie qu'il ne pourra pas atteindre la fin de l'itinéraire, et il faut donc revoir à la baisse certains objectifs, notamment les objectifs de vitesse de parcours de l'itinéraire.

En revanche, sur un itinéraire très court par exemple, et pour rester dans les limites de sécurité (notamment une fréquence cardiaque inférieure à $FC_{Max}$) il peut arriver que toute l'énergie consommable ne puisse pas être consommée sur l'itinéraire.

Sur la figure 4, le sujet a déjà parcouru une portion de l'itinéraire (Xo à $X_t$).

A l'étape F41, à un instant t, le dispositif acquiert sa position et réalise une comparaison 85 des valeurs 33 mesurées (vitesse mesurée Vm, fréquence cardiaque mesurée FCm) de $X_0$ à $X_t$ aux objectifs (VO$_1$, FCO$_1$) établis avant le début de l'effort physique.

L'étape F42 représente la mesure de la vitesse Vm du sujet de $X_0$ à $X_t$, celle-ci est nécessaire au calcul de l'énergie consommée par le sujet, réalisé à l'étape F45.

De même, l'étape F43 représente la mesure de la fréquence cardiaque FCm du sujet de $X_0$ à $X_t$, celle-ci est nécessaire au calcul des valeurs 34 prévisionnelles de la fréquence cardiaque (FCP), réalisé à l'étape F44.

Dans l'exemple de la figure 4, le sujet est allé plus vite que programmé (Vm est au-dessus de VO$_1$), et sa fréquence cardiaque est en conséquence beaucoup plus haute que programmée (FCm au-dessus de FCO$_1$), bien qu'elle reste inférieure à $FC_{Max}$ entre $X_0$ et $X_t$.

Au cours de l'étape F44, à partir des données enregistrées (Vm, FCm) entre $X_0$ et $X_t$, des valeurs 35 programmées à long-terme de vitesse (VO1) de course, et de la topologie de l'itinéraire à venir, le dispositif produit des valeurs 34 prévisionnelles à court-terme de la fréquence cardiaque (FCP) sur un horizon de temps $\Delta$.

Au court de l'étape F441, ces valeurs 34 prévisionnelles à court-terme (FCP) sont comparées 84 aux valeurs 44 limites prédéterminées de fréquence cardiaque pour ce sujet ($FC_{min}$ et $FC_{Max}$), et la comparaison 84 indique que la fréquence cardiaque va dépasser la valeur limite $FC_{Max}$ ($FCP \geq FC_{Max}$). De sorte que les conditions de la comparaison 84 ne sont pas remplies (842), et l'unité de traitement de données du dispositif émet un message de conseil 52 de ralentissement à l'utilisateur par l'intermédiaire, par exemple, de l'écran 99.

A l'étape F441, l'une des conditions ($FC_{min} \leq FC \leq FC_{Max}$ ; $FC_{min} \leq FCP \leq FC_{Max}$) n'étant pas respectée, l'étape F46 est amorcée (chemin 446).

A l'étape F45, la vitesse Vm du sujet étant plus élevée que programmé sur la première portion parcourue de l'itinéraire, le dispositif calcule que l'énergie réellement consommée ECr est plus importante que la prévision ECO$_1$, et donc l'énergie réellement restante QEr diminue plus rapidement. Donc si le sujet maintient une vitesse supérieure ou égale aux valeurs 35 programmées, il ne sera pas en mesure d'atteindre $X_f$ puisque sa réserve en énergie consommable sera nulle avant qu'il n'atteigne $X_f$.

Un nouveau profil de valeurs 35 programmées doit être établi pour éviter le dépassement de $FC_{Max}$, et pour permettre au sujet de conserver de l'énergie jusqu'à $X_f$.

Pour calculer l'énergie consommée en fonction de la vitesse, de la topographie, et/ou des paramètres environnementaux (vent par exemple), le dispositif portatif d'assistance à la gestion de l'effort physique utilise par exemple dans le cas d'un coureur, une équation enregistrée dans une mémoire 42, du type (équation (2)) :

$$Re.\int_{t=T}^{t=T+\Delta} (P_{roulement} + P_{dénivelé} + P_{air}).t.dt = E(\omega)$$

En effet, à tout instant, le bilan des forces s'exerçant sur le sujet s'écrit :

$$m.a = F_{roulement} + F_{dénivelé} + F_{air}$$

Avec : a, l'accélération instantanée du sujet

$$F_{roulement} = m.g.Cr$$

$$F_{dénivelé} = m.g.\sin(p(t))$$

$$F_{air} = \tfrac{1}{2}.\rho(t).S.Cx.V(t)^2$$

d'où avec la relation $P(t) = \vec{F}(t).\vec{V}(t)$

$$P_{roulement} = m.g.V(t).Cr$$

$$P_{dénivelé} = m.g.V(t).\sin(p(t))$$

$$P_{air} = \tfrac{1}{2}.\rho(t).S.Cx.V(t)^3$$

Où V(t) est la vitesse du sujet en valeur absolue à un instant t, $\Delta$ est la durée écoulée depuis un instant T sur laquelle on réalise le calcul, c'est-à-dire l'horizon de temps des prévisions, et E($\Delta$) est l'énergie dépensée par le sujet pendant la durée $\Delta$.

Les coefficients m (masse), Cr (coefficient de frottement propre du coureur (de ses semelles) et à l'état de la route), S (surface apparente), Cx (coefficient de trainée) et Re (rendement énergétique) peuvent être personnalisés à un sujet particulier. L'utilisateur peut pour cela entrer manuellement lesdits coefficients. Alternativement ou en complément, ces coefficients propres au sujet peuvent être affinés automatiquement par le dispositif après chaque session d'effort physique réalisée par le sujet. Le dispositif ajuste ces coefficients en inversant le calcul précédemment effectué et à partir des vitesses effectivement mesurées lors du parcours de l'itinéraire par le sujet. Les coefficients p(t) (pente moyenne à un instant t), g (accélération de la pesanteur) et $\rho$(t) (densité de l'air à l'instant t correspondant à un point x d'altitude donnée) sont des données 41 enregistrées en mémoire 4.

A l'étape F46, le dispositif établi donc un nouveau profil de valeurs 35 programmées de la vitesse ($VO_2$), plus modeste, qui sera le profil de vitesse de référence pour le reste de l'itinéraire.

Puis, à l'étape F47, le dispositif établi un nouveau profil de valeurs 35 programmées de la fréquence cardiaque $FCO_2$ en se basant sur le nouveau profil $VO_2$, plus modeste, qui sera le profil de vitesse de référence pour le reste de l'itinéraire.

Puis le dispositif renouvelle l'étape F441 afin de vérifier que le nouveau profil de vitesse $VO_2$ est compatible avec une fréquence cardiaque $FCO_2$ comprise dans la fourchette de $FC_{min}$ et $FC_{Max}$.

Les étapes F46, F47, F441 sont renouvelées dans cet ordre jusqu'à ce que les critères appliqués à l'étape F441 soient remplis. Le dispositif passe ensuite (chemin 448) à l'étape F48.

A l'étape F48, le dispositif établi un nouveau profil de valeurs 35 programmées de l'énergie consommée $ECO_2$ à partir notamment du nouveau profil $VO_2$, qui sera le profil de vitesse de référence pour le reste de l'itinéraire. Il établi aussi un nouveau profil de valeurs 35 programmées de la quantité d'énergie consommable restante $QEO_2$ à partir des valeurs $ECO_2$.

Puis, à l'étape F481, le dispositif vérifie qu'aucune des valeurs 35 programmées de la quantité d'énergie $QEO_2$ n'est égale à zéro avant que le sujet n'atteigne $X_f$. Si la quantité d'énergie restante programmée atteint zéro avant la position $X_f$, le dispositif établi un nouveau profil de vitesse compatible avec ce critère (passage à l'étape F46 par le chemin 486).

Si le critère de l'étape F481 est satisfait, le dispositif termine cette suite d'étapes (chemin 480).

Le dispositif renouvelle les étapes F41 à F481 à intervalles de temps prédéterminés.

Les autres paramètres mesurés permettent d'affiner les calculs de fréquence cardiaque et d'énergie consommée. Ainsi, la saturation en dioxygène du sang influe sur la performance physique, et donc sur la fréquence cardiaque. De même, la température du corps ou l'état d'hydratation du sujet influent sur la performance physique. Par exemple, une perte hydrique de 1% mène aux premiers signes de diminution de la performance.

Les paramètres environnementaux tels que température extérieure et pression atmosphérique influent aussi sur des paramètres tels que la fréquence cardiaque et la saturation en dioxygène du sang, et ils sont à l'origine de modifications de la performance (cf. par exemple « Précis de physiologie de l'exercice musculaire », Per-Olof Åstrand et Kaare Rodahl, Editions Masson, 3e ed., 1994). Ainsi, par exemple, l'altitude (pression partielle en dioxygène faible) limite la performance. De même, une température élevée agit comme majorant de la consommation énergétique.

Par ailleurs, un tel dispositif selon l'invention dispose de moyens 70 de communication avec un dispositif extérieur. De tels moyens 70 de communication peuvent permettre au dispositif d'être relié à un ordinateur personnel qui permet de charger des fichiers dans un sens ou dans un autre entre le dispositif et l'ordinateur. Par exemple des historiques de données et des profils d'utilisateurs peuvent être téléchargés du dispositif vers l'ordinateur, et des données relatives à un itinéraire (cartes, topologie, conditions météorologiques, etc.) peuvent être téléchargées de l'ordinateur vers le dispositif.

L'invention peut faire l'objet de nombreuses autres variantes de réalisation non représentées.

En particulier, l'ensemble des équations présentées résultent d'études biologiques, biomécaniques, et/ou physiologiques, et elles ne sont citées qu'à titre d'exemples. Toute autre équation jugée plus appropriée où plus exacte peut être utilisée dans un dispositif selon l'invention. Elles peuvent par exemple être mises en oeuvre par des réseaux neuronaux.

De même les étapes logiques présentées le sont à titre d'exemple d'un mode de réalisation particulier et elles peuvent être réalisées dans des ordres logiques différents.

De plus les paramètres physiologiques mesurés peuvent être choisis en fonction du sujet, du type d'effort (type de sport, rééducation, compétition, etc.) et peuvent inclure d'autres paramètres jugés utiles mais non présentés parmi les exemples cités ci-avant. Les paramètres d'effort dont un dispositif selon l'invention est apte à prévoir l'évolution à plus ou moins court-terme peuvent aussi être plus nombreux que ceux présentés.

Par ailleurs, rien n'empêche, dans un dispositif selon l'invention, que de nombreux autres paramètres environnementaux déterminants pour l'effort physique pratiqué soient pris en compte : état de surface d'un terrain, dangerosité, etc.

De plus, un dispositif selon l'invention étant destiné à être utilisé en extérieur, et éventuellement pour des efforts physiques tels que de la natation, il est avantageusement imperméable à l'eau.

## Revendications

1. Dispositif (1) portatif d'assistance à la gestion d'un effort physique fourni par un sujet comprenant :

   - au moins un capteur (10, 11, 12) d'un paramètre physiologique, dit paramètre d'effort, évoluant en fonction de l'effort physique, ledit capteur étant apte à fournir des données numériques, dites données d'effort, représentatives dudit paramètre d'effort,
   - au moins un dispositif, dit dispositif (20) de positionnement, apte à déterminer sa propre position dans un référentiel topographique donné et à fournir des données numériques, dites données de positionnement, représentatives de cette position,
   - au moins une interface (98, 99) de communication avec un utilisateur,
   - au moins une mémoire (4) dans laquelle sont susceptibles d'être enregistrées des données, dites données d'entrées, comprenant au moins :

     o des données de positionnement fournies par ledit dispositif de positionnement,
     o des données d'effort,
     o des données représentatives d'un itinéraire à parcourir dans ledit référentiel topographique par ledit sujet au cours de l'effort physique,

   **caractérisé en ce que** le dispositif comprend :

   - au moins une unité (30) de traitement de données, agencée pour :

     ▪ produire des données, dites données prévisionnelles, représentatives de l'évolution d'au moins un paramètre d'effort sur l'itinéraire restant à parcourir par le sujet, en fonction des données d'entrées, à partir d'équations et de tableaux de valeurs enregistrés dans au moins une mémoire du dispositif,

- comparer ces données prévisionnelles avec des valeurs prédéterminées enregistrées dans au moins une mémoire dudit dispositif d'assistance,
- produire des données représentatives d'un message dont le contenu est fonction du résultat de la comparaison des données prévisionnelles et des valeurs prédéterminées,
- transmettre lesdites données représentatives d'un message à l'interface en vue de la communication dudit message à un utilisateur.

2. Dispositif selon la revendication 1, **caractérisé en ce qu'**un premier paramètre d'effort est la fréquence cardiaque et au moins un second paramètre d'effort est choisi parmi : la saturation en dioxygène du sang, la température corporelle, le taux de glucose dans le sang, la tension artérielle, la température corporelle, la fréquence respiratoire, la conductivité de la peau.

3. Dispositif selon l'une des revendications 1 ou 2, **caractérisé en ce que** ladite unité (30) de traitement de données est agencée pour produire des données prévisionnelles représentatives de l'évolution des paramètres d'effort en fonction des données d'entrée.

4. Dispositif selon l'une des revendications 1 à 3, **caractérisé en ce que** ladite unité (30) de traitement de données est agencée pour produire au moins une donnée prévisionnelle représentative d'une dépense énergétique en fonction des données d'entrée et de données représentatives de l'évolution d'au moins un paramètre d'effort.

5. Dispositif selon l'une des revendications 1 à 4, **caractérisé en ce que** ladite unité (30) de traitement de données est agencée pour comparer chaque paramètre d'effort à au moins une valeur, dite valeur seuil, prédéterminée et propre audit paramètre d'effort.

6. Dispositif selon l'une des revendications 1 à 5, **caractérisé en ce qu'**il comporte des moyens d'alarme activés par au moins une donnée prévisionnelle d'au moins un paramètre d'effort en dehors d'une fourchette de valeurs prédéterminées.

7. Dispositif selon l'une des revendications 1 à 6, **caractérisé en ce qu'**il comporte au moins un capteur (13, 14) environnemental apte à fournir des données, dites données environnementales, relatives à l'environnement du dispositif.

8. Dispositif selon la revendication 7, **caractérisé en ce que** l'unité (30) de traitement de données est agencée pour produire des données prévisionnelles au moins partiellement dépendantes desdites données environnementales.

9. Dispositif selon l'une des revendications 1 à 8, **caractérisé en ce qu'**il est adapté pour, après chaque effort, modifier et enregistrer dans une mémoire (4) au moins un profil d'utilisateur constitué d'au moins une valeur utile à au moins une équation utilisée par l'unité (30) de traitement de données pour produire des données prévisionnelles.

10. Dispositif selon l'une des revendications 1 à 9, **caractérisé en ce qu'**il est agencé pour produire des données prévisionnelles pour au moins deux types d'effort physique distincts.

11. Dispositif selon l'une des revendications 1 à 10, **caractérisé en ce qu'**il dispose d'au moins un moyen (70) de communication.

12. Dispositif selon l'une des revendications 1 à 10, **caractérisé en ce qu'**il comprend en outre un accéléromètre.

13. Dispositif selon la revendication 12, **caractérisé en ce que** l'accéléromètre est embarqué sur le sujet.

14. Dispositif selon la revendication 12, **caractérisé en ce que** l'accéléromètre est embarqué à bord d'un véhicule piloté par le sujet.

15. Dispositif selon l'une des revendications 12 à 14, **caractérisé en ce que** l'unité (30) de traitement de données est agencée pour produire des données prévisionnelles en fonction de données d'entrées comprenant des données de positionnement comprenant au moins pour partie des données fournies par ledit accéléromètre.

16. Dispositif selon l'une des revendications 12 à 15, **caractérisé en ce que** l'unité (30) de traitement de données est agencée pour produire des données prévisionnelles en fonction de données d'entrées comprenant, à titre de données de positionnement, uniquement des données fournies par ledit accéléromètre.

17. Dispositif selon l'une des revendications 12 à 16, **caractérisé en ce que** les signaux délivrés par ledit accéléromètre servent à évaluer le niveau de fatigue du sujet.

**Patentansprüche**

1. Tragbare Hilfsvorrichtung (1) zur Verwaltung einer körperlichen Anstrengung, die von einem Lebewesen aufgebracht wird, umfassend:

   - zumindest einen Sensor (10, 11, 12) eines physiologischen Parameters, der Anstrengungsparameter genannt wird, und sich in Abhängigkeit von der körperlichen Anstrengung verändert, wobei der besagte Sensor in der Lage ist, numerische Daten beizusteuern, die Anstrengungsdaten genannt werden, und repräsentativ für den besagten Anstrengungsparameter sind,
   - zumindest eine Vorrichtung, die Positionierungsvorrichtung (20) genannt wird, und die in der Lage ist, seine eigene Position in einem gegebenen topographischen Bezugsrahmen zu bestimmen, und numerische Daten beizusteuern, die Positionierungsdaten genannt werden, und repräsentativ für diese Position sind,
   - zumindest eine Schnittstelle (98, 99) zur Kommunikation mit einem Benutzer,
   - zumindest einen Speicher (4), in dem Daten, die Eingangsdaten genannt werden, gespeichert werden können, welche zumindest folgendes umfassen:
   - Positionierungsdaten, die von der besagten Positionierungsvorrichtung beigesteuert werden,
   - Anstrengungsdaten,
   - Repräsentative Daten für eine Route, die vom besagten Lebewesen im Zuge der körperlichen Anstrengung im besagten topographischen Bezugsrahmen zurückzulegen ist,

   **dadurch gekennzeichnet, dass** die Vorrichtung folgendes umfasst:

   - zumindest eine Einheit (30) zur Datenverarbeitung, die eingerichtet ist, um:
   - Daten zu erzeugen, die voraussichtliche Daten genannt werden, und repräsentativ für die Entwicklung zumindest eines Anstrengungsparameters auf der verbleibenden Route ist, die je nach Eingangsdaten vom Lebewesen zurückzulegen ist, ausgehend von Gleichungen und Tabellen mit Werten, die in zumindest einem Speicher der Vorrichtung gespeichert sind,
   - diese voraussichtlichen Daten mit den vorbestimmten Werten zu vergleichen, die in zumindest einem Speicher der besagten Hilfsvorrichtung gespeichert sind,
   - repräsentative Daten für eine Meldung zu erzeugen, deren Inhalt vom Ergebnis des Vergleichs der voraussichtlichen Daten mit den vorbestimmten Werten abhängt,
   - die besagten repräsentativen Daten für eine Meldung an die Schnittstelle zu übertragen, um die besagte Meldung einem Benutzer zugänglich zu machen.

2. Vorrichtung nach Anspruch 1, **dadurch gekennzeichnet, dass** ein erster Anstrengungsparameter die Herzfrequenz ist, und zumindest ein zweiter Anstrengungsparameter unter den folgenden ausgewählt wird: die Disauerstoffsättigung im Blut, die Körpertemperatur, der Glukoseanteil im Blut, der Blutdruck, die Körpertemperatur, die Atemfrequenz, die Leitfähigkeit der Haut.

3. Vorrichtung nach einem der Ansprüche 1 oder 2, **dadurch gekennzeichnet, dass** die besagte Einheit (30) zur Datenverarbeitung eingerichtet ist, um in Ahängigkeit von den Eingangsdaten repräsentative voraussichtliche Daten über die Entwicklung der Anstrengungsparameter zu erzeugen.

4. Vorrichtung nach einem der Ansprüche 1 bis 3, **dadurch gekennzeichnet, dass** die besagte Einheit (30) zur Datenverarbeitung eingerichtet ist, um in Ahängigkeit von den Eingangsdaten zumindest ein repräsentatives voraussichtliches Datum für einen Energieverbrauch und repräsentative Daten für die Entwicklung zumindest eines Anstrengungsparameters zu erzeugen.

5. Vorrichtung nach einem der Ansprüche 1 bis 4, **dadurch gekennzeichnet, dass** die besagte Einheit (30) zur Datenverarbeitung eingerichtet ist, um jeden Anstrengungsparameter mit zumindest einem Wert, der Grenzwert genannt wird, zu vergleichen, der vorbestimmt und charakteristisch für den besagten Anstrengungsparameter ist.

6.  Vorrichtung nach einem der Ansprüche 1 bis 5, **dadurch gekennzeichnet, dass** sie Alarmeinrichtungen umfasst, die durch zumindest ein voraussichtliches Datum zumindest eines Anstrengungsparameters ausgelöst werden, das sich außerhalb einer vorbestimmten Wertespanne befindet.

7.  Vorrichtung nach einem der Ansprüche 1 bis 6, **dadurch gekennzeichnet, dass** sie zumindest einen Umgebungssensor (13, 14) umfasst, der in der Lage ist, Daten in Bezug auf die Umgebung der Vorrichtung beizusteuern, die Umgebungsdaten genannt werden.

8.  Vorrichtung nach Anspruch 7, **dadurch gekennzeichnet, dass** die Einheit (30) zur Datenverarbeitung eingerichtet ist, um voraussichtliche Daten zu erzeugen, die zumindest teilweise von den besagten Umgebungsdaten abhängig sind.

9.  Vorrichtung nach einem der Ansprüche 1 bis 8, **dadurch gekennzeichnet, dass** sie eingestellt ist, um nach jeder Anstrengung zumindest ein Benutzerprofil in einem Speicher (4) zu verändern und abzuspeichern, das aus zumindest einem für zumindest eine Gleichung nützlichen Wert gebildet wird, die von der Einheit (30) zur Datenverarbeitung verwendet wird, um voraussichtliche Daten zu erzeugen.

10. Vorrichtung nach einem der Ansprüche 1 bis 9, **dadurch gekennzeichnet, dass** sie eingerichtet ist, um voraussichtliche Daten für zumindest zwei unterschiedliche körperliche Anstrengungstypen zu erzeugen.

11. Vorrichtung nach einem der Ansprüche 1 bis 10, **dadurch gekennzeichnet, dass** sie über zumindest eine Kommunikationseinrichtung (70) verfügt.

12. Vorrichtung nach einem der Ansprüche 1 bis 10, **dadurch gekennzeichnet, dass** sie darüber hinaus einen Beschleunigungsmesser enthält.

13. Vorrichtung nach Anspruch 12, **dadurch gekennzeichnet, dass** der Beschleunigungsmesser vom Lebewesen getragen wird.

14. Vorrichtung nach Anspruch 12, **dadurch gekennzeichnet, dass** der Beschleunigungsmesser an Bord eines Fahrzeugs angebracht ist, das vom Lebewesen gesteuert wird.

15. Vorrichtung nach einem der Ansprüche 12 bis 14, **dadurch gekennzeichnet, dass** die Einheit (30) zur Datenverarbeitung eingerichtet ist, um in Abhängigkeit von den Eingangsdaten, die Positionierungsdaten enthalten, die zumindest teilweise Daten enthalten, die vom besagten Beschleunigungsmesser beigesteuert werden, voraussichtliche Daten zu erzeugen.

16. Vorrichtung nach einem der Ansprüche 12 bis 15, **dadurch gekennzeichnet, dass** die Einheit (30) zur Datenverarbeitung eingerichtet ist, um in Abhängigkeit von den Eingangsdaten, die als Positionierungsdaten nur Daten enthalten, die vom besagten Beschleunigungsmesser beigesteuert werden, voraussichtliche Daten zu erzeugen.

17. Vorrichtung nach einem der Ansprüche 12 bis 16, **dadurch gekennzeichnet, dass** die vom besagten Beschleunigungsmesser beigesteuerten Daten dazu dienen, den Ermüdungszustand des Lebewesens zu bewerten.

**Claims**

1.  Portable device (1) for assisting the management of a physical exertion provided by a subject, comprising:

    - at least one sensor (10, 11, 12) for a physiological parameter, called exertion parameter, evolving as a function of the physical exertion, said sensor being suitable to provide digital data, called exertion data, that are representative of said exertion parameter,
    - at least one device, called positioning device (20), that is suitable to determine its own position in a given topographical frame of reference and to provide digital data, called positioning data, that are representative of this position,
    - at least one interface (98, 99) for communication with a user,
    - at least one memory (4) in which data, called input data, are capable of being recorded, comprising at least:

o positioning data provided by said positioning device,
o exertion data,
o data that are representative of an itinerary to be travelled in said topographical frame of reference by said subject in the course of the physical exertion,

wherein the device comprises:

- at least one data-processing unit (30), designed:

    ▪ to generate data, called forecast data, that are representative of the evolution of at least one exertion parameter over the itinerary remaining to be travelled by the subject, as a function of the input data, from equations and from tables of values recorded in at least one memory of the device,
    ▪ to compare these forecast data with predetermined values recorded in at least one memory of said assistance device,
    ▪ to generate data that are representative of a message, the content of which is a function of the result of the comparison of the forecast data and the predetermined values,
    ▪ to transmit said data that are representative of a message to the interface, with a view to the communication of said message to a user.

2. Device as claimed in Claim 1, wherein a first exertion parameter is the heart rate and at least one second exertion parameter is chosen from: the oxygen saturation of the blood, the body temperature, the level of glucose in the blood, the blood pressure, the respiratory rate, the conductivity of the skin.

3. Device as claimed in one of Claims 1 or 2, wherein said data-processing unit (30) is designed to generate forecast data that are representative of the evolution of the exertion parameters as a function of the input data.

4. Device as claimed in one of Claims 1 to 3, wherein said data-processing unit (30) is designed to generate at least one forecast datum that is representative of an expenditure of energy as a function of the input data and of data that are representative of the evolution of at least one exertion parameter.

5. Device as claimed in one of Claims 1 to 4, wherein said data-processing unit (30) is designed to compare each exertion parameter to at least one predetermined value, called threshold value, that is specific to said exertion parameter.

6. Device as claimed in one of Claims 1 to 5, including alarm means activated by at least one forecast datum of at least one exertion parameter outside a range of predetermined values.

7. Device as claimed in one of Claims 1 to 6, including at least one environmental sensor (13, 14) that is suitable to provide data, called environmental data, relating to the environment of the device.

8. Device as claimed in Claim 7, wherein the data-processing unit (30) is designed to generate forecast data that are at least partly dependent on said environmental data.

9. Device as claimed in one of Claims 1 to 8, wherein the device is suitable to modify and record in a memory (4), after each exertion, at least one user profile constituted by at least one value that is useful for at least one equation used by the data-processing unit (30) in order to generate forecast data.

10. Device as claimed in one of Claims 1 to 9, wherein the device is designed to generate forecast data for at least two distinct types of physical exertion.

11. Device as claimed in one of Claims 1 to 10, wherein the device has at least one means (70) of communication.

12. Device as claimed in one of Claims 1 to 10, wherein the device further comprises an accelerometer.

13. Device as claimed in Claim 12, wherein the accelerometer is worn by the subject.

14. Device as claimed in Claim 12, wherein the accelerometer is on-board a vehicle piloted by the subject.

**15.** Device as claimed in one of Claims 12 to 14, wherein the data processing unit (30) is designed to generate forecast data as a function of the input data comprising positioning data comprising at least partly data provided by said accelerometer.

**16.** Device as claimed in one of Claims 12 to 15, wherein the data processing unit (30) is designed to generate forecast data as a function of input data comprising, as positioning data, only data provided by said accelerometer.

**17.** Device as claimed in one of Claims 12 to 16, wherein the signals delivered by said accelerometer are used to assess the subject level of fatigue.

Fig 1

Fig 2

# Fig 3

Fig 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7534206 B

**Littérature non-brevet citée dans la description**

- A dynamic heart rate model for training optimization in cycling (P83). **ANKANG LE ; THOMAS JAITNER ; FRANK TOBIAS ; LOTHAR LITZ.** The Engineering and Sport. vol. 7

- **JACK H. WILMORE ; DAVID L. COSTILL ; ARLETTE DELAMARCHE ; PAUL DELAMARCHE ; CAROLE GROUSSARD.** *Physiologie du sport et de l'exercice : Adaptations physiologiques à l'exercice physique,* 2006
- **PER-OLOF ÅSTRAND ; KAARE RODAHL.** Précis de physiologie de l'exercice musculaire. 1994